# EUROPEAN PATENT APPLICATION

(11) **EP 1 918 293 A1**
(43) Date of publication of application: **07.05.2008**
(21) Application number: 06121651.1
(22) Date of filing: 02.10.2006
(51) Int. Cl.: C07D 495/04

(54) **Process for the preparation of 6-substituted-4-oxo-thieno [2,3b]thiopyran-2-sulfonic acid**

(71) Applicant: SANDOZ AG, 4056 Basel (CH)
(72) Inventor: The designation of the inventor has not yet been filed
(74) Representative: Dressel, Jürgen

(57) **Abstract**

The present invention relates to one-pot process for the preparation of 6-substituted-4-oxo-thieno[2,3b]thiopyran-2-sulfonic acid of formula (I), which comprises, reacting 2-thienylthiol with alkenyl acid in the presence of an aromatic hydrocarbon solvent and a base, cyclization of the resulting compound and further reacting with an acid anhydride and sulfuric acid to get the desired compound of formula (I).

The compound of formula (I) is the key intermediate in the preparation of (S,S)-5,6-dihydro-4-alkyylamino-6-substituted-4H-thieno[2,3-b]thiopyran-2-sulfonamide-7,7-dioxide and analogs thereof.

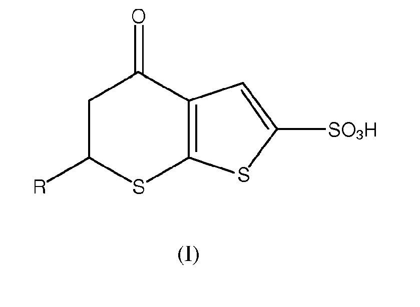

## Description

### Field of the Invention

The present invention relates to a process for the preparation of 6-substituted-4-oxo-thieno[2,3b]thiopyran-2-sulfonic acid of formula (I). 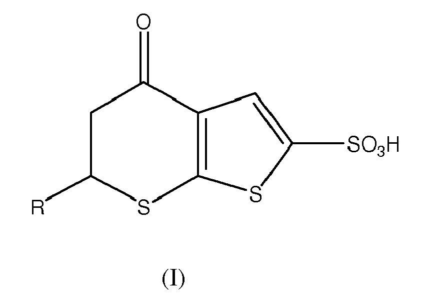 wherein R is C₁₋₆ straight or branched chain alkyl, C₁₋₆ substituted alkyl or C₁₋₆ alkoxy-C₁₋₆ alkyl.

The compound of formula (I) is a key intermediate in the preparation of the corresponding sulfonamide compound of formula (II) and its analogs thereof 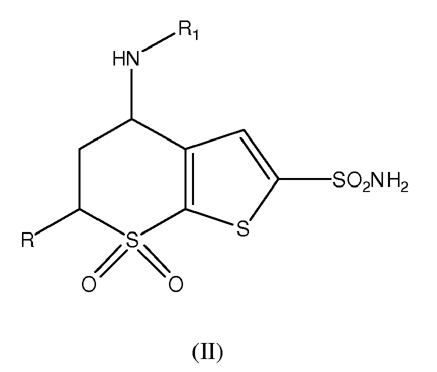 wherein R and R₁ are individually, C₁₋₆ straight or branched chain alkyl, C₁₋₆ substituted alkyl or C₁₋₆ alkoxy-C₁₋₆ alkyl.

The compound of formula (II), especially dorzolamide, wherein R is methyl and R₁ is ethyl, is useful in the treatment of glaucoma, which is characterized by elevated intraocular pressure.

### Background of the Invention

U.S. Patent No. 4,797,413 (the '413 patent) discloses several carbonic anhydrase inhibitors including dorzolamide. The '413 patent also discloses a process for the preparation of dorzolamide and its analogue and comprises reacting 2-thiophene thiol with a substituted acrylic acid to form the corresponding alkyl 3-(thien-2-ylthio)butyrate. The process further comprises cyclizing the obtained alkyl 3-(thien-2-ylthio)butyrate, in the presence of, oxalylchloride and stannous chloride, to the corresponding thieno[2,3b] thiopyran-4-one. Thieno[2,3b] thiopyran-4-one is next converted to compound of formula (II), which involves several successive steps. This compound of formula (II) is a diastereomeric compound, the isomers of which must be separated and resolved to obtain the most active (S,S)-enantiomer.

U.S.Patent Nos. 4,968,815 and 4,968,814 both disclose a process for the preparation of chiral (S)-(3-thien-2-ylthio)butyric acid, which is the key intermediate in the preparation of dorzolamide.

U.S.Patent No. 5,760,249 discloses a process for the preparation of hydroxysulfone, which is a key intermediate in the preparation of dorazolamide.

These prior art processes, however, involve many steps utilizing multiple solvents, require use of hazardous raw materials like SnCl₄ (Friedal craft reaction), trifluoro acetic anhydride, oxalylchloride which are expensive and time consuming.

Accordingly, there is a need for a process for the synthesis of compound of formula (I) which is more economical than available process while eliminating use of heavy metal oxidants.

### Summary of the invention

The present invention relates to a process for the preparation of 6-substituted-4-oxo-thieno[2,3b]thiopyran-2-sulfonic acid of formula (I), which is a key intermediate in the preparation of (S,S)-5,6-dihydro-4-alkyylamino-6-substituted-4H-thieno[2,3-b]thiopyran-2-sulfonamide-7,7-dioxide (compound of formula, II) and analogs thereof.

Specifically, the present invention relates to a one-pot process for the preparation of a compound of formula (I), which comprises reacting 2-thiophenethiol with alkenyl acid in the presence of an aromatic hydrocarbon solvent and an organic base, cyclizing the resulting compound and further reacting with an acid anhydride and sulfuric acid to get the final compound of formula (I).

The process of the present invention, for the preparation of compound of formula (I), reduces the known multi-step procedures to a single-step reaction (one-pot process) and eliminates the use of hazardous raw materials.

According to the present invention, process for the preparation of compound of formula (I) is represented in scheme-1. 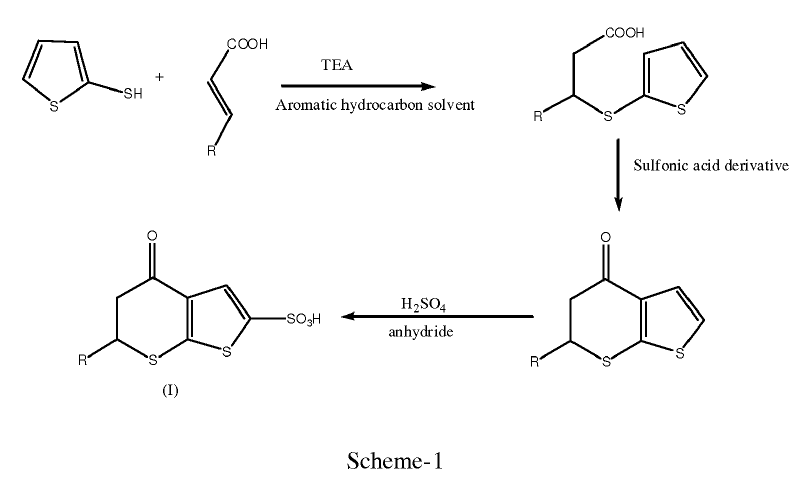 wherein R is C₁₋₆ straight or branched chain alkyl, C₁₋₆ substituted alkyl or C₁₋₆ alkoxy-C₁₋₆ alkyl.

### Detailed Description of the Invention

The present invention relates to a process for the preparation of a compound of formula (I) by a one-pot process using 2-thiophenethiol as the starting material. The compound of formula (I) is a key intermediate in the preparation of the corresponding sulfonamide compound of formula (II) and its analogs thereof.

Specifically, the process of the present invention relates to a one-pot process for the preparation of a compound of formula (I), which is obtained by reacting 2-thiophenethiol with alkenyl acid, followed by cyclizing the resulting compound to form the corresponding thieno[2,3b]thiopyran-4-one, which on further reaction with sulfuric acid in the presence of an acid anhydride, provides the compound of formula (I).

According to the present invention, the process for the preparation of a compound of formula (1) comprises the steps of ;
a) adding 2-thiophenethiol, to the solution of an organic base and alkenyl acid in an aromatic hydrocarbon solvent;
b) stirring the contents of step (a) at a temperature about 50-75°C for about 25 hours;
c) adding the reaction mixture of step (b) to the solution of an anhydrous sulfonic acid derivative and an aromatic hydrocarbon solvent, at about room temperature to reflux temperature or vice versa;
d) stirring the reaction solution of step (c) at reflux temperature for about 2-6 hours;
e) quenching the reaction mixture of step (d) by addition of water or base, while stirring;
f) separating the aqueous layer from the reaction solution of step (e);
g) extracting the aqueous layer of step (f) with an aromatic hydrocarbon solvent;
h) combining the organic extractions of step (g) and drying over sodium sulfate;
i) adding an acid anhydride, to the solution of step (h) while stirring;
j) adding sulfuric acid to the reaction mixture of step (i) at -5 to 0°C;
k) stirring the contents of step (j) at about 0 to 5°C for about 2-5 hours to separate a solid;
l) filtering of the separated solid of step (k) under reduced pressure; and
m) drying the solid of step (1) under reduced pressure at about 50-65°C;

The organic base used in step (a) comprises lower alkyl primary amines like methyl amine, lower alkyl secondary amines like diethyl amine, lower alkyl tertiary amines like triethyl amine, aromatic amines, such as benzyl amines, and mixtures thereof.

The aromatic hydrocarbon solvent used in above reaction comprises benzene, toluene or xylene. In one embodiment the aromatic hydrocarbon solvent is toluene.

The sulfonic acid derivative used in step (c) includes but is not limited to, alkyl sulfonic acids (such as methane sulfonic acid, propyl sulfonicacid and hexyl sulfonic acid) and aryl sulfonic acids (such as para toluene sulfonic acid and benzyl sulfonic acid).

The acid anhydride used in step (i) includes but is not limited to propionic anhydride, butanoic anhydride and benzoic anhydride.

In one embodiment of the present invention the one-pot process produces a compound of formula (I) wherein R is methyl.

In another aspect of the present invention, the compound of formula (I), wherein R is methyl is further converted to a compound of formula (II), wherein R is methyl and R₁ is ethyl i.e. dorzolamide.

In another embodiment of the present inventin, the process of conversion of compound of formula (I), wherein R is methyl to a compound of formula (II), wherein R is methyl and R₁ is ethyl i.e. dorzolamide comprises,
a) reacting the compound of formula (I) obtained by the process as described above with an halogenating agent in the presence of halogenated hydrocarbon solvent and isolating the corresponding halogenated compound;
b) reacting the compound obtained in step (a) with ammonium hydroxide to get the corresponding amide derivative;
c) converting the carbonyl group in compound obtained in step (b) to the corresponding hydroxyl group by reductive reaction;
d) reacting the compound obtained in step (c) with oxone to get the corresponding sulfone dioxide compound;
e) reacting the obtained compound of step (d) with acetonitrile followed by reduction gives the corresponding alkyl amine derive;
f) converting the compound obtained in step (e) to the corresponding acid addition salt; and
g) separating the diastereomers (cis:trans isomers) from the diastereomeric mixture of compound of step (f) followed by resolution to get the compound of formula (II) and its pharmaceutically acceptable salts.

The halogenating agent used in step (a) selected from PCl₅, PCl₃ or thionyl chloride.

The acid addition salt of step (f) is inorganic acid addition salt or organic acid addition salt, wherein the inorganic acid addition salt is hydrochloric acid, hydrobromic acid, nitric acid or sulfuric acid addition salt and the organic acid addition salt is phthalic acid, adipic acid, oxalic acid, maleic acid, fumaric acid, malic acid, salicylic acid or acetic acid.

The process of the present invention further explains in details by the following examples which are not limited to the scope of the invention.

### EXAMPLES

### Example: 1

### Preparation of 5,6-dihydro-4-oxo-6-methyl-4H-thieno[2,3b]thiopyran-2-sulfonic acid

15 grams of 2-thiophenethiol was added to the solution of 6.5 grams of triethyl amine, 11.8 grams of crotonic acid and 45 ml of toluene. The contents were stirred at 60-65°C for about 3 hours. The reaction mixture was cooled and charged into an anhydrous solution of 111 grams of para toluenesulfonic acid in 2500 ml of toluene at reflux temperature. Continued the stirring at reflux temperature and removed water azeotropically for about 3 hours. 400 ml of water was added to the reaction mixture at 25-30°C. The aqueous layer was separated, and extracted with 25ml of toluene. The combined toluene layers were dried over sodium sulfate, and filtered. 51.7 grams of propionic anhydride was added to the above toluene reaction mixture. 13.6 gm of concentrated sulfuric acid was added to the reaction mixture at -5 to 0°C. The reaction mass stirred for 3 hours at 0 to 5°C. The separated solid was filtered and washed with 25 ml of toluene. The solid was dried under reduced pressure at abo ut 60°C.

(Yield: 17.7 grams; Purity: 96.4% by HPLC)

### Example: 2

### Preparation of 5,6-dihydro-4-oxo-6 -methyl-4H-thieno[2,3b]thiopyran-2-sulfonic acid

50 grams of 2-thiophenethiol was added to the solution of 21.7 grams of triethyl amine, 39.2 grams of crotonic acid and 150 ml of toluene. The contents were stirred at 60-65°C for about 3 hours. The reaction mixture was cooled to 0-5°C and to it was charged the preheated (75-80°C) anhydrous solution of 370.2 grams of para toluenesulfonic in 720 ml of toluene. Heated the reaction mixture to reflux. Continued the stirring at refux temperature and removed water azeotropically for about 5 hours. 275 ml of triethyl amine and 1300 ml of water was added to the reaction mixture at 25-30°C. The aqueous layer was separated, and extracted with 500 ml of toluene. The combined toluene layers were distilled to recover 700 ml of toluene at 50-55°C under reduced pressure. 172.2 grams of propionic anhydride was added to the above reaction mixture. 45.5 gm of concentrated sulfuric acid was added to the reaction mixture at -5 to 0°C. The reaction mass stirred for 3 hours at 0 to 5°C. The separated solid was filtered and washed with 300 ml of toluene. The solid was dried under reduced pressure at about 60°C.

(Yield: 69 grams ; Purity: 95% by HPLC)

### Example: 3

### Preparation of 3-(2-mercaptothiophene)butanoic acid.

500 grams of 2-thiophenethiol was added to the solution of 217.3 grams of triethyl amine, 392.3 grams of crotonic acid and 1500 ml of toluene. The reaction mixture was heated to 60-65°C and stirred for 3 hours. The toluene was distilled off from the reaction mixture under reduced pressure, to get the crude containing the title compound.

(Yield: 998 grams; Purity: 90% by HPLC)

### Example: 4

### Preparation of 3-(2-mercaptothiophene)butanoic acid.

20 grams of 2-thiophene thiol was added to the mixture of 12.88 grams of crotonic acid, and 11.84 ml of triethyl amine. The reaction mixture was heated to 60-65°C and stirred for 3 hours. 2 grams of crotonic acid was added to the reaction mixture and stirred for 3.5 hours at 60-65°C. A solution of 8 ml concentrated hydrochloric acid in 10 ml of water was added to the reaction mixture at 25-30°C. The reaction mixture was extracted with ethyl acetate (2x30ml). The combined ethyl acetate hyer was dried, and distilled under reduced pressure to get the crude compound containing the title compound.

(Yield: 31 grams; Purity: 87.5 % by HPLC)

### Example: 5

### Preparation of 5,6-dihydro-4-oxo-6-methyl-4H-thieno[2,3b]thiopyran

11.89 grams of methanesulfonic acid was added into 200 ml of toluene and heated azeotropically at 110-115°C for about 30minutes to remove water traces. A solution of 5 grams of 3-(2-mercaptothiophene)butanoic acid in 50 ml of toluene was added at reflux for about 30 minutes. The reaction mixture was stirred for 3 hours at reflux. 100 ml of water was added to the reaction mixture at 25-30°C. The aqueous layer was separated, and extracted with 25 ml of toluene. The combined toluene layers were dried and toluene was distilled under reduced pressure to get the title compound as crude product.

(Yield: 2.84 grams; Purity: 83.67% by HPLC)

### Example: 6

### Preparation of 5,6-dihydro-4-oxo-6-methyl-4H-thieno[2,3b]thiopyran

212.9 grams of para toluenesulfonic acid was added into 550ml of toluene and heated at 110-115°C for about 30minutes to remove water azeotropically. A solution of 50 grams of 3-(2-mercaptothiophene)butanoic acid in 50 ml of toluene was added to the above reaction mixture at reflux over a period of 40 minutes. The reaction mixture was stirred for 3 hours at reflux. 2500 ml of water was added to the reaction mixture at 25-30°C. The aqueous layer was separated, and extracted with 500ml of toluene. The combined toluene layers were dried, and toluene was distilled under reduced pressure to get the title compound as crude product..

(Yield: 29.5 grams; Purity: 87.9 % by HPLC).

### Example: 7

### Preparation of 5,6-dihydro-4-oxo-6 -methyt-4H-thieno[2,3b]thiopyran-2-sulfonic acid

108.9 grams of propionic anhydride was added to the mixture of 50 grams of 5,6-dihydro-4-oxo-6-methyl-4H-thieno[2,3b]thiopyran and 150 ml of toluene at 25-30°C. 28.7 grams of concentrated sulfuric acid was added to the reaction mixture at -5 to 0°C. The reaction mass stirred for 3 hours at -5 to 5°C. The separated solid was filtered and washed with 2x30m1 of toluene. The solid was dried under reduced pressure at about 60°C.

(Yield: 47 grams; Purity: 95.4% by HPLC)

### Example: 8

### Preparation of 5,6-Dihydro-4H-6-methyltheino[2,3,b]thippyran-4-one-2-Sulfonyl chloride.

To a suspension of product from Example 2 (200 g, 0.77 mol) in CH₂Cl₂ (975 ml), cooled to -8°C was added the suspension of PCl₅ (474 g, 2.27 mol) in CH₂Cl₂ (7.5 ltr) dropwise maintaining the temperature below 0°C. The mixture was stirred at 0°C for ½ hour and then poured into ice water (1.5 ltr). The layers were separated, the aqueous phase further extracted with and CH₂Cl₂ and the organic layers were washed with water. Drying and solvent evaporation gave the title compound (224 g).

### Example 9:

### Preparation of 5,6-Dihydro-4H-6-methyttheino[2,3,b]thiopyran-4-one -2-Sulfonamide.

To ammonium hydroxide (586 ml), cooled to -30°C was added a solution of product from Example 8 (224 g, 0.77 mol) in acetone (770 ml) dropwise, maintaining the temperature between -20°C and -30°C. The mixture was stirred at 0°C for 1 hour, concentrated, the solid collected, washed with water and dried to give the title compound (175 g.).

### Example 10:

### Preparation of 5,6-Dihydro-4H-4-hydroxy-6-methyltheino[2,3,b]thiopyran-2-sulfonamide.

To a suspension of product from Example 9 (100.0 g,0.38mol) in absolute ethanol (1000 ml) was added sodium borohydride (17.5 g, 0.46mol). The mixture was refluxed for 2 hrs and then stirred at room temperature overnight. The mixture was cooled to 0°C, acidified with 1N HCl, basified with sodium bicarbonate solution, concentrated and extracted with ethyl acetate. Solvent evaporation gave the title compound (86 g,).

### Example 11:

### Preparation of 5,6-Dihydro-4H-4-hydroxy-6-methyltheino[2,3,b]thiopyran-2-sulfonamide-7,7-dioxide.

To a suspension of product from Example 10 (26.8 g,0.10 mol) in methanol (500 ml) was added a solution of oxone (104.5g,0.17 mol) in water (567 ml) dropwise and the resulting suspension was stirred at room temperature overnight. The mixture was concentrated, diluted with water and extracted with ethyl acetate. Drying and solvent evaporation gave the product as a solid (23. g).

### Example 12:

### Preparation of 5,6-Dihydro-4H-4-ethylamino-6-methyltheino[2,3,b]thiopyran-2-sulfonamide-7,7 -dioxide (mixture of Cis and Trans)

To a solution of product from Example 11 (37 g, 0.12 mol) in CH₃CN (555 ml) cooled to -10°C was added dropwise 95.5% H₂SO₄ (132 ml). After the addition, the suspension was allowed to warm to room temperature. After stirring overnight, the solution was poured onto ice and stirred for 1 hour. The solution was then extracted with ethyl acetate and the organic extracts were backwashed with NaHCO₃, dried, filtered and concentrated to dryness to yield 30.0 g of 5,6-Dihydro-4H-4-acetylamino-6-methyltheino[2,3,b]thiopyran-2- sulfonamide-7,7-dioxide.

A suspension of the acetylamino compound (8.8 g, 0.026 mol) in THF (180 ml) was fitted with a short path distillation head and the mixture was heated at reflux while a solution of borane-dimethylsulfide (9.0 ml, 0.09 mol) was added dropwise with stirring. After 1.5 hours, the reaction mixture was concentrated to dryness, the residue treated with 12N HCl, and the mixture heated at reflux. After 0.5 hour, the suspension was concentrated to dryness, and the residue was treated with NaHCO₃. The mixture was extracted with ethyl acetate and the organic layers were dried, filtered and concentrated to dryness to yield 7.1 g of a mixture of trans- and cis-isomers.

## Claims

1. A process for the preparation of compound of formula (I) 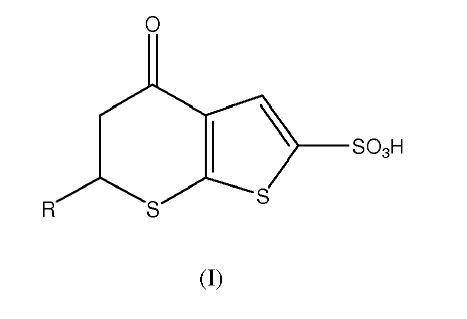 wherein R is C₁₋₆ straight or branched chain alkyl, C₁₋₆ substituted alkyl or C₁₋₆ alkoxy-C ₁₋₆ alkyl.
which comprises,
(a) adding 2-thiophenethiol, to the solution of an organic base, alkenyl acid and an aromatic hydrocarbon solvent;
(b) stirring the contents of step (a) at a temperature about 50-75°C for about 2-5 hours;
(c) adding of the reaction mixture of step (b) to the solution of an anhydrous sulfonic acid derivative and an aromatic hydrocarbon solvent at about room temperature to reflux temperature and vice versa;
(d) stirring the reaction solution of step (c) at reflux temperature for about 2-6 hours;
(e) quenching the reaction mixture of step (d) by addition of water or a base such as triethyl amine, aqueous sodium carbonate while stirring;
(f) separating the aqueous layer from the reaction solution of step (e);
(g) extracting the aqueous layer of step (f) with an aromatic hydrocarbon solvent;
(h) combining the organic extractions of step (g) and drying over sodium sulfate;
(i) adding of an acid anhydride, to the reaction solution of step (h) while stirring;
(j) adding of sulfuric acid to the reaction mixture of step (i) at 5 to 0°C;
(k) stirring the contents of step (j) at about 0 to -5°C for about 2-5 hours to separate a solid;
(l) filtering of the separated solid of step (k) under reduced pressure; and
(m) drying the solid of step (1) under reduced pressure at about 50-65°C;

2. The process according to claim 1, preparation the compound of formula (I) wherein R is methyl.

3. The process according to claim 1, wherein the aromatic hydrocarbon solvent selected from benzene, toluene or xylene.

4. The process according to claim 2, wherein the aromatic hydrocarbon solvent is toluene.

5. The process according to claim 1, wherein the sulfonic acid derivative is an alkyl sulfonic acid or an aryl sulfonic acid

6. The process according to claim 5, wherein the alkyl sulfonic acid is methane sulfonic acid, propanesulfonic acid or hexanesulfonic acid.

7. The process according to claim 6, wherein the alkyl sulfonic acid is methane sulfonic acid.

8. The process according to claim 5, wherein an aryl sulfonic acid is para toluene sulfonic acid or benzene sulfonic acid.

9. The process according to claim 8, wherein an aryl sulfonic acid is para toluene sulfonic acid.

10. The process according to claim 1, wherein the acid anhydride is propionic anhydride, butanoic anhydride or benzoic anhydride.

11. The process according to claim 10, wherein the acid anhydride is propionic anhydride.

12. The process according to claim 1, wherein R is methyl, the aromatic hydrocarbon solvent is toluene, the sulfonic acid derivative is para toluenesulfonic acid and the acid anhydride is propionic anhydride.

13. The process for the preparation of compound of formula (II) 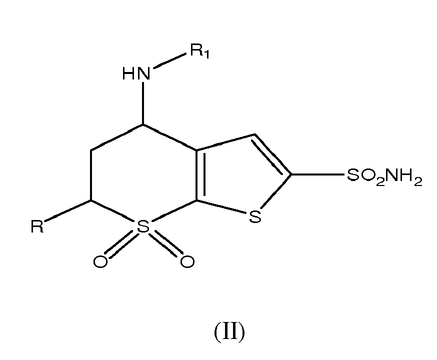 wherein R and R₁ are individually, C₁₋₆ straight or branched chain alkyl, C₁₋₆ substituted alkyl or C₁₋₆ alkoxy-C₁₋₆ alkyl.
which comprising the steps of ;
(a) reacting the compound of formula (I) obtained by the process of claim 1 with an halogenating agent in the presence of halogenated hydrocarbon solvent and isolating the corresponding halogenated compound;
(b) reacting the compound obtained in step (a) with ammonium hydroxide to get the corresponding amide derivative;
(c) converting the carbonyl group in compound obtained in step (b) to the corresponding hydroxyl group by reductive reaction;
(d) reacting the compound obtained in step (c) with oxone to get the corresponding sulfone dioxide compound;
(e) reacting the obtained compound of step (d) with acetonitrile followed by reduction gives the corresponding alkyl amine derive;
(f) converting the compound obtained in step (e) to the corresponding acid addition salt; and
(g) separating the diastereomers (cis:trans isomers) from the diastereomeric mixture of compound of step (f) followed by resolution to get the compound of formula (II) and its pharmaceutically acceptable salts.

14. The claim of 13, wherein the halogenating agent in step (a) selected from PC15, PC13 or thionylchliride.

15. The claim of 13, wherein the acid addition salt is inorganic acid or organic acid addition salt.

16. The claim of 15, wherein the inorganic acid is hydrochloric acid, sulfuric acid, nitric acid or hydroromic acid.

17. The claim of 15, wherein the organic acid is phthalic acid, adipic acid, oxalic acid, maleic acid, fumaric acid, malic acid, salicylic acid or acetic acid.

18. The claim of 16, wherein the inorganic acid is hydrochloric acid.
